(19) Europäisches Patentamt
European Patent Office
Office européen des brevets

(11) Publication number: **0 255 219 B1**

(12) # EUROPEAN PATENT SPECIFICATION

(45) Date of publication of patent specification: **11.03.92**

(51) Int. Cl.5: **C09K 19/12**, C09K 19/08, C09K 19/30, C07C 69/157, C07C 69/24, C07D 239/26, C07D 239/34, C07D 213/30, C07D 213/65, C07D 237/06, C07D 241/18

(21) Application number: **87305098.3**

(22) Date of filing: **09.06.87**

The file contains technical information submitted after the application was filed and not included in this specification

(54) **Omega-substituted, optically active 2-alkanol ester derivatives.**

(30) Priority: **09.06.86 JP 133269/86**
**23.03.87 JP 68629/87**

(43) Date of publication of application:
**03.02.88 Bulletin 88/05**

(45) Publication of the grant of the patent:
**11.03.92 Bulletin 92/11**

(84) Designated Contracting States:
**CH DE FR GB LI**

(56) References cited:
**EP-A- 0 131 373**
**EP-A- 0 168 043**

**CHEMICAL ABSTRACTS SERVICE, REGISTRY HANDBOOK, NUMBER SECTION, 1965-1971, Registry nos. 4600-00-4 through 12699-98-8, page 2400R, column 1, lines 39, 43RN: 5437-02-5, 5437-06-9**

(73) Proprietor: **Chisso Corporation**
**6-32, Nakanoshima 3-chome Kita-ku**
**Osaka-shi Osaka-fu(JP)**

(72) Inventor: **Saito, Shinichi**
**10-1, Otsutomocho Kanazawaku**
**Yokohamashi Kanagawaken(JP)**
Inventor: **Inukai,Takashi**
**4-46,Mori 3-chome Isogoku**
**Yokohamashi Kanagawaken(JP)**
Inventor: **Inoue, Hiromichi**
**10-2, Otsutomocho Kanazawaku**
**Yokohamashi Kanagawaken(JP)**
Inventor: **Miyazawa, Kazutoshi**
**12-14, Mutsuura 2-chome Kanazawaku**
**Yokohamashi Kanagawaken(JP)**
Inventor: **Ohno, Kouji**
**10-3, Otsutomocho Kanazawaku**
**Yokohamashi Kanagawaken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 255 219 B1

Inventor: **Ushioda, Makoto**
**12-16, Kannon 1-chome Kawasakiku**
**Kawasakishi Kanagawaken(JP)**

(74) Representative: **Lamb, John Baxter et al**
**MARKS & CLERK 57-60 Lincoln's Inn Fields**
**London WC2A 3LS(GB)**

## Description

BACKGROUND OF THE INVENTION

1. Field of the Invention

This invention relates to a novel organic compound and a liquid crystal composition containing the same, and more particularly it relates to an organic compound having an optically active group and useful as a component of ferroelectric liquid crystal compositions and a ferroelectric liquid crystal composition containing the same.

2. Description of the Related Art

At present, TN (Twisted Nematic) type display mode has been broadly employed for liquid crystal display elements. This TN liquid crystal display has many advantages such as lower driving voltage, less power consumption, etc. However, the display element is inferior in the aspect of response speed to emissive type display elements such as cathode ray tube, electroluminescence, plasma display, etc. A new TN type display element having the twist angle increased to a range of 180° to 270° has also been developed, but it is still inferior in the aspect of response speed. As described above, various attempts for improvement have been made, but they have not yet been brought to practical use. Recently, however, a novel display mode using ferroelectric liquid crystals has been extensively researched, and according to such a mode, there is a possibility that the response speed is notably improved (Clark et al, Applied Phys. lett., 36, 899 (1980)). This mode is directed to a method of making use of chiral smectic phases such as chiral smectic C phase (hereinafter abbreviated to SC*) exhibiting ferroelectricity. Phases exhibiting ferroelectricity are not only SC* phase, alone,but it is known that chiral smectic phases of F, G, H, I, etc. also exhibit ferroelectricity.

Many specific features are required for ferroelectric liquid crystal materials used for ferroelectric liquid crystal display elements utilized practically, but at present there is no single compound which satisfies the requirements; hence it is necessary to use a ferroelectric liquid crystal composition obtained by blending some liquid crystals or non-crystalline liquid crystal compounds.

Further, not only a ferroelectric liquid crystal composition consisting only of ferroelectric liquid crystal compounds, but also a ferroelectric liquid crystal composition has been proposed in Japanese patent application laid-open No. Sho 61-19518, which composition is obtained by blending at least one compound exhibiting a ferroelectric liquid crystal compound with compound(s) or a composition exhibiting an achiral smectic C, F, G, H, I or the like (hereinafter abbreviated to SC, SF, SG, SH, SI, etc., respectively) phase as basic substance(s) to give a ferroelectric liquid crystal composition as a whole. Still further, a ferroelectric liquid crystal composition has been reported in Mol. Cryst. Liq. Cryst., 89, 327 (1982), which composition is obtained by blending at least one compound which is optically active but exhibits no ferroelectric liquid crystalline phase with compound(s) or a composition exhibiting SC phase or the like as a main component to give a ferroelectric liquid crystal composition as a whole.

As a summary of these facts, it is seen that when at least one optically active compound irrespective of whether or not this compound exhibits a ferroelectric liquid crystalline phase, is blended with smectic phaseexhibiting liquid crystal substance(s), it is possible to constitute a ferroelectric liquid crystal composition. Nevertheless, it is preferred that such an optically active substance also exhibit a liquid crystalline phase, and even when it exhibits no liquid crystalline phase, it is preferred to be a compound the structure of which is similar to those of liquid crystal compounds, so to speak a pseudo-liquid crystal substance.

SUMMARY OF THE INVENTION

The present inventors have found a compound characterized by increasing the spontaneous polarization value Ps as one of the important specific features required for the ferroelectric liquid crystal composition, and have attained the present invention.

The present invention resides in a compound expressed by the formula

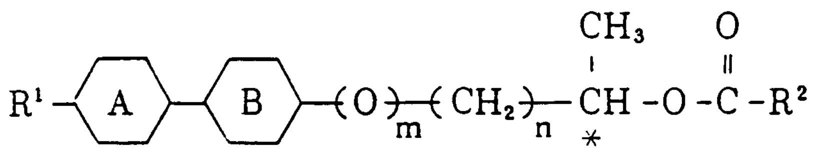 (I)

wherein $R^1$ represents a linear or branched chain alkyl group, alkoxy group, alkanoyl group, alkanoyloxy group, alkoxycarbonyl group, alkoxycarbonyloxy group or alkoxyalkyl group, each of 1 to 20 carbon atoms; $R^2$ represents hydrogen atom or a linear or branched chain alkyl group, alkoxy group or alkyl group substituted at $\omega$-position thereof by halogen atom, cyano group, thioalkyl group, alkoxy group, alkanoyl group, alkanoyloxy group, alkoxycarbonyl group or alkoxycarbonyloxy group, each of 1 to 15 carbon atoms;

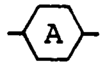 and 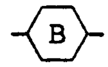

each independently represent

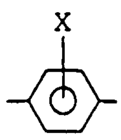, 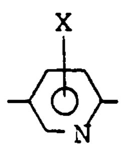, 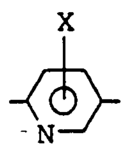, 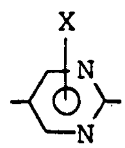, 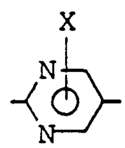, 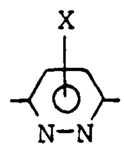,

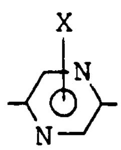 or 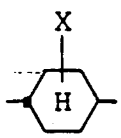

wherein X represents hydrogen atom, F atom, Cℓ atom, CN group, methyl group or trifluoromethyl group; m represents 0 or 1; n represents a natural number of 1 to 10; and the symbol * indicates that the carbon atom onto which the symbol * is attached is an asymmetric carbon atom, and a ferroelectric liquid crystal composition containing the same.

DETAILED DESCRIPTION OF PREFERRED EMBODIMENTS

Preferred examples of

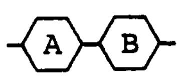

in the formula (I) are

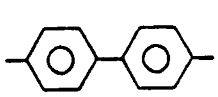, 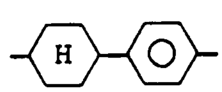, 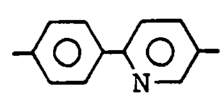, 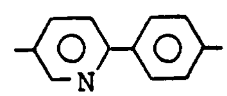,

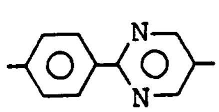, 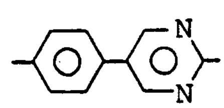, 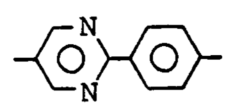, 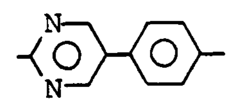,

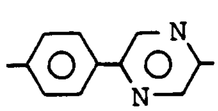, 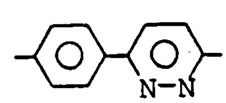,

F-substitute, CN group-substitute and methyl group-substitute thereof, etc.

Preferred examples of $R^1$ are the groups as defined above but having 4 to 15 carbon atoms. When $R^1$ is a branched chain group and the presence of optivally active form is possible, $R^1$ may be either in the optically active from or of course in the racemic form.

More preferred examples of $R^1$ are a linear or branched chain alkyl group or alkoxy group of 6 to 12 carbon atoms, and those which are linear or methyl group-branched are particularly preferred.

The $\omega$-substituted alkyl group in $R^2$ refers to a group expressed by the formula $-Y-CH_2-Z$ wherein Y represents a single bond or an alkylene group and Z represents a halogen atom, cyano group, a thioalkyl group, an alkoxy group, an alkanoyl group, an alkanoyloxy group, an alkoxycarbonyl group or an alkoxycarbonyloxy group.

Preferred examples of $R^2$ are an alkyl group, an alkoxy group and an $\omega$-substituted-alkyl group each of 1 to 8 carbon atoms, and when $R^2$ is an branched chain group and the presence of optically active form is possible, such a group may be those in optically active form and these often have superior specific features to those of a linear chain group. A more preferred example of $R^2$ is an alkyl group. m is preferably 1, n is preferably in the range of 1 to 6, more preferably 1 to 4.

The compound of the present invention itself is not always liquid crystalline, but when it is used as a component of ferroelectric liquid crystal compositions, it is possible to increase the spontaneous polarization value Ps of the compositions. Here, the importance of the spontaneous polarization Ps will be briefly described. In general, the response time $\tau$ in ferroelectric liquid crystal display elements is expressed by the following formula (II)

$$\tau = \frac{\eta}{Ps \cdot E} \qquad (II)$$

wherein $\eta$ represents viscosity and E represents electric field strength.

As seen from the formula (II), reduction in the response time is effected by increasing Ps or reducing the viscosity.

The compound of the present invention, when used as a component of ferroelectric liquid crystal compositions, has a function of notably increasing the Ps of the ferroelectric liquid crystal compositions and as a result it is possible to reduce the response time. As described later in Examples, for example when a compound of the formula (I) of the present invention is added in 20% by weight to a liquid crystal composition exhibiting an achiral smectic C phase (which composition exhibits no spontaneous polarization), a ferroelectric liquid crystal composition having a large spontaneous polarization value is formed, and the response time exhibited by this composition is as short as 50 $\mu$ sec. at 25°C (Example 5). Further, when a compound of the formula (I) of the invention is added to a composition exhibiting chiral smectic C phase but having a very small Ps, it is possible to raise Ps up to a practical value (Example 6). In short, it can be said that the compound of the present invention is much suprior as the one contributing to improvement in Ps.

Further, since the compound of the formula (I) of the present invention has an optically active carbon atom, when it is added to a nematic liquid crystal, it has a capability of inducing a twisted structure in the resulting composition. Nematic liquid crystals having a twisted structure i.e. chiral nematic liquid crystals do not form the so-called reverse domain (dechiralization lines); hence it is possible to use the compound of the formula (I) as an agent for preventing the reverse domain from forming.

Further, when the compound of the present invention is added to a nematic liquid crystal composition, the resulting chiral nematic liquid crystal composition has a very flat or negative chiral pitch temperature characteristic, as shown in Example 9 mentioned later. As to most of the chiral substances currently used for adding to namatic liquid crystals, the higher the temperature, the longer the chiral pitch, whereas substances having such a characteristic that the higher the temperature, the shorter the chiral pitch have also been reported, and it has been known that such substances reduce the temperature dependency of the threshold voltage as an electrooptical characteristic of TN type display elements (see 33rd Applied Physics-Related Associated Lecture Meeting (1986, spring), Collected Lecture Preliminary Manuscripts, Ip-G-7 (page 78) and JAPAN DISPLAY '86, Collected Lecture Preliminary Manuscripts, 8.3 (pages 286 -289)).

Since the compound of the present invention has physical properties similar to those of the above substances, it is possible to reduce the temperature- dependency of the threshold voltage of a chiral nematic composition having the compound blended therein.

Further, apart therefrom, in the case of the socalled super TN type display where the twist angle is

180°-270° in the TN type displays, change of pitch due to temperature variation notably reduces the display grade, whereas in the case where a chiral nematic liquid crystal composition having the compound of the present invention blended therein is used in the super TN type display, it is possible to prepare an excellent super TN type display element according to which the display grade is not damaged by temperature change.

As described above, the compound of the present invention is also useful as a chiral component compound of chiral nematic compositions.

Here, a relationship between the compound of the present invention and a compound disclosed in Japanese patent application laid-open No. Sho 61-44845 as a prior application will be referred to. The above prior application discloses a general formula (I) including an astronomically large number of compounds, and the compound of the present invention might be formally included in the formula (I), but the specification of the prior application does not disclose the same compounds as those of the present invention not only concretely, but also in the form of divided formulas of the formula (I).

Further, the effectiveness of the compounds of the prior application aimed therein consists in that the compounds are added to a nematic liquid crystal to induce the cholesteric phase in the resulting composition and thereby improve the temperature characteristics of its pitch. Thus the above effectiveness has nothing to do with the ferroelectric liquid crystals aimed in the present invention. Accordingly, it can be said that the compound of the present invention is not disclosed in the above-mentioned prior application.

Next, the preparation of the compound of the formula (I) will be described below.

The compound of the formula (I) may be prepared for example as illustrated in the following equations, using an ω-substituted-optically active 2-alkanol as an intermediate:

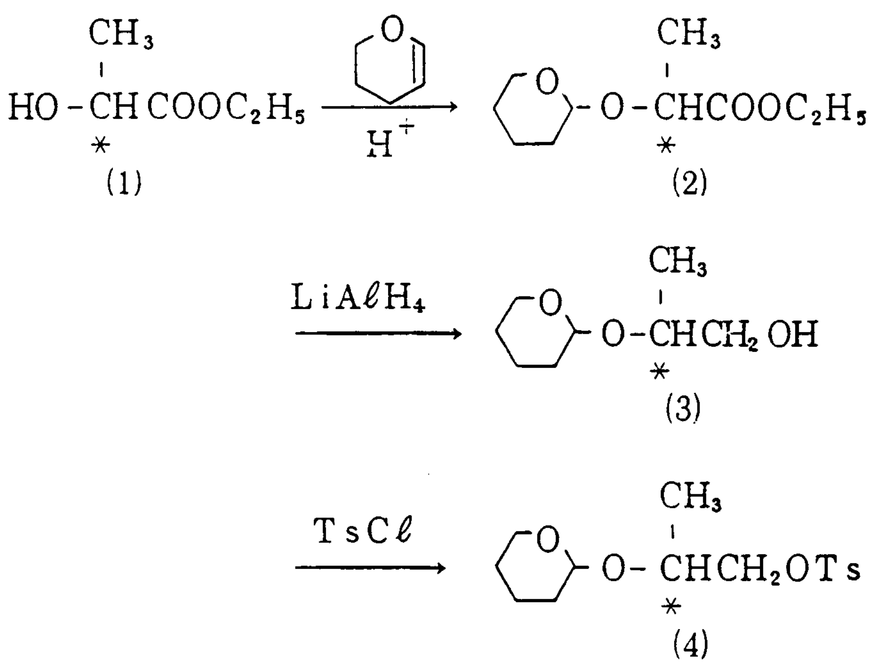


(a) Wehn m = 0 and n = 1,

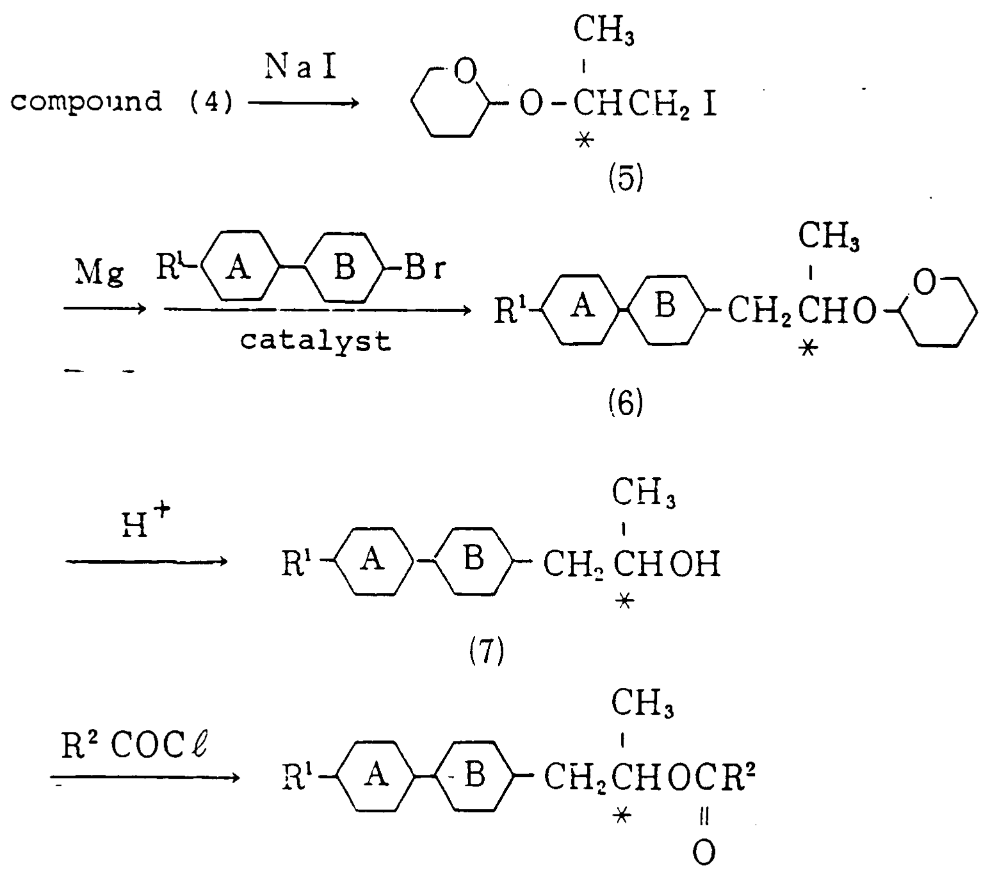


(5)

(6)

(7)

(I)

Namely, compound (4) is reacted with NaI to obtain 1-iodo-2-(2-tetrahydropyranyloxy)-propane (5) which is then converted into a Grignard reagent, followed by coupling to obtain a compound (6) which is subjected to removal of the protecting group (hereinafter referred to as "deprotection") to obtain an ω-substituted-2-alkanol (7) which is esterified to obtain the objective compound of the formula (I).

(b) When m = 0 and n = 2,

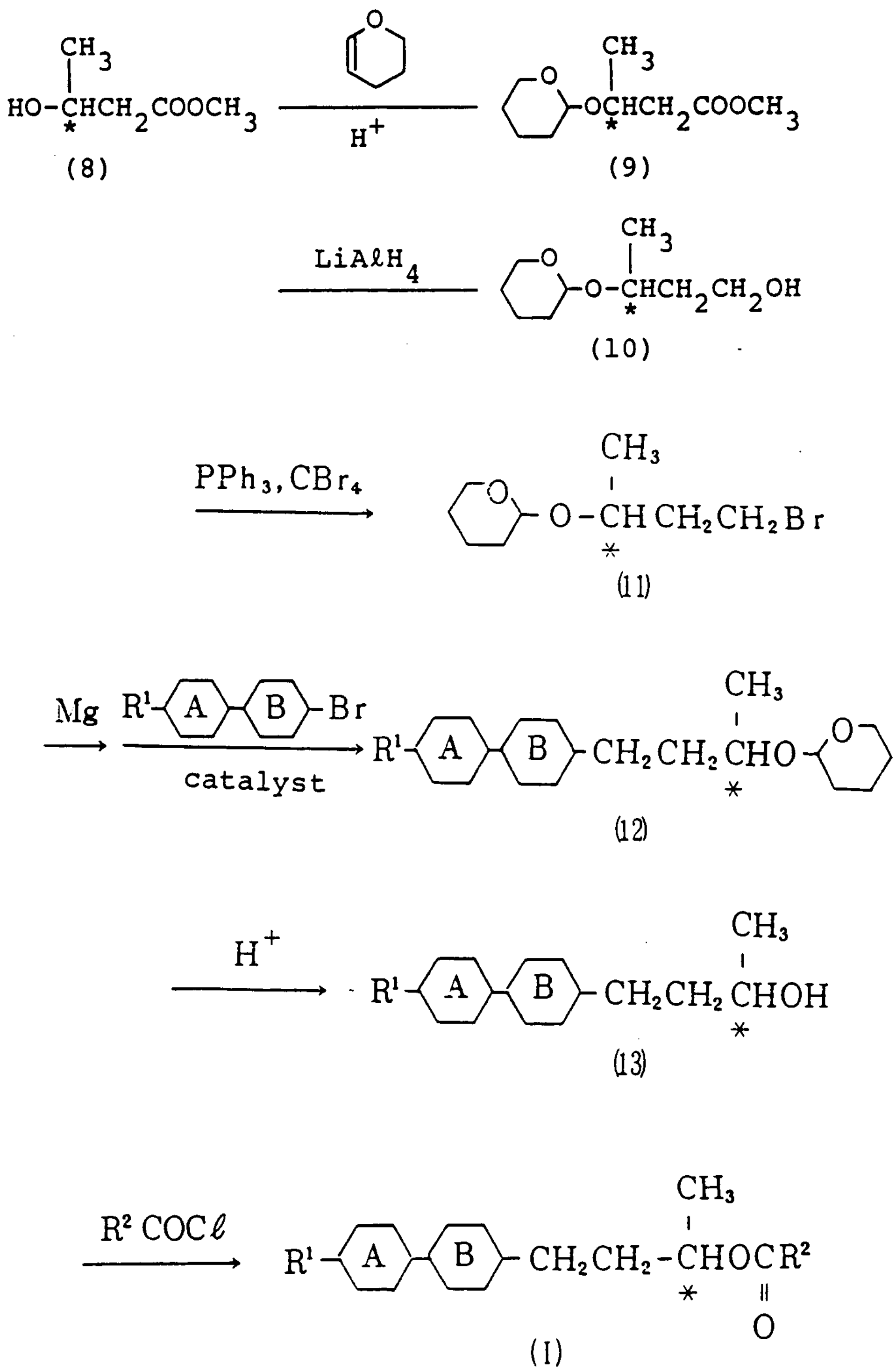


(8) (9) (10) (11) (12) (13) (I)

Namely, dihydropyran is reacted with methyl 3-hydroxy-butanoate (8) to obtain a tetrahydropyranyl compound (9) with which a reducing agent such as lithium aluminum hydride is reacted to obtain an alcohol (10) which is subjected to Br-substitution to obtain 1-bromo-3-(2-tetrahydropyranyloxy)-butane (11), which is converted into a Grinard reagent, followed by coupling to obtain a compound (12) which is subjected to deprotection to obtain an ω-substituted-2-alkanol (13) which is esterified to obtain the objective compound (I).

(c) When m = 0 and n ≧ 3,

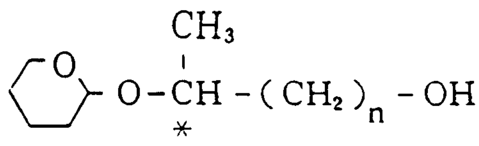

(14)

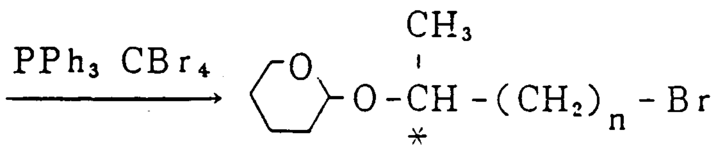


(15)

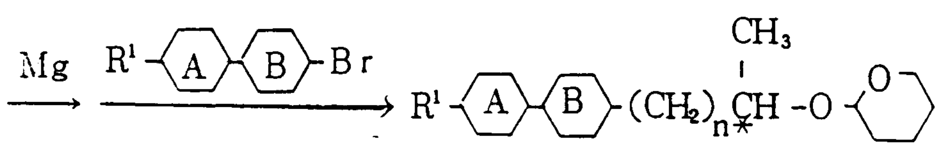


(16)

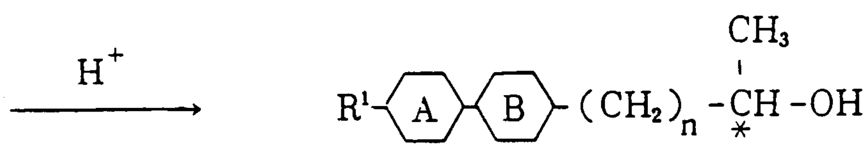


(17)

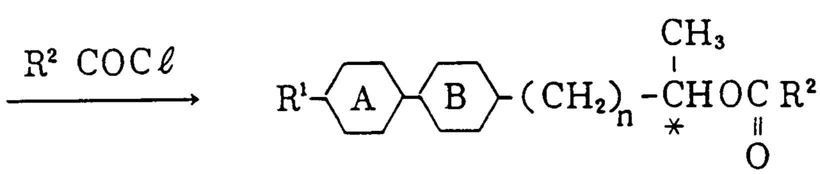


(I)

Namely, an alcohol (14) is brominated into a compound (15) which is subjected to coupling reaction to obtain a compound (16) which is deprotected and then esterified to obtain the objective compound (I).
(d) When m = 1,

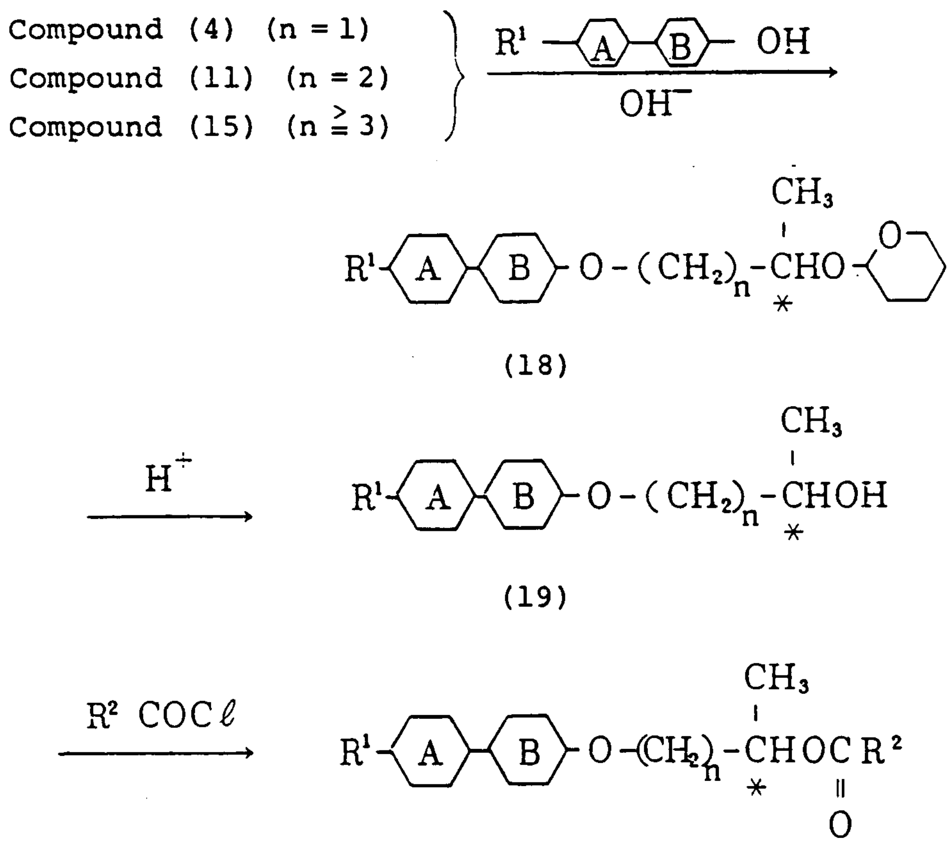


(18)

(19)

(I)

Namely, the respective raw materials, i.e. compound (4) in the case of n = 1, compound (11) in the case of n =2 or compound (15) in the case of n ≧ 3 are reacted with a phenol for etherification to obtain the corresponding compounds (18) which are deprotected to obtain the corresponding ω-substituted-2-alkanols (19) which are esterified to obtain the objective compounds (I).

Concrete representative examples of compounds of the formula(I) of the present invention obtained as above are as follows:

4′-pentyl-4-(2′-acetoxypropoxy)-biphenyl
4′-pentyl-4-(2′-propanoyloxypropoxy)-biphenyl
4′-pentyl-4-(2′-butanoyloxypropoxy)-biphenyl (S form, m.p. 50.8 -51.8 ° C)
4′-pentyl-4-(2′-pentanoyloxypropoxy)-biphenyl (S form, m.p. 54.9 -55.9 ° C)
4′-pentyl-4-(2′-hexanoyloxypropoxy)-biphenyl (S form, m.p. 53.8 - 59.8 ° C)
4′-pentyl-4-(2′-heptanoyloxypropoxy)-biphenyl (S form, m.p. 52.4 - 53.0 ° C)
4′-pentyl-4-(2′-octanoyloxypropoxy)-biphenyl
4′-pentyl-4-(2′-nonanoyloxypropoxy)-biphenyl
4′-pentyl-4-(2′-(3″-methyl-pentanoyloxy)-propoxy)-biphenyl
4′-heptyl-4-(2′-pentanoyloxypropoxy)-biphenyl (S form, m.p. 59.1 - 60.3 ° C)
4′-heptyl-4-(2′-hexanoyloxypropoxy)-biphenyl (S form, m.p. 66.6 - 68.6 ° C)
4′-heptyl-4-(2′-octanoyloxypropoxy)-biphenyl (S form, m.p. 56.6 - 57.3 ° C)
4′-heptyl-4-(2′-(5″-methyl-heptanoyloxy)-propoxy)-biphenyl (2′S,5″S,m.p. 57.5 - 60.6 ° C)
4′-octyl-4-(2′-propanoyloxypropoxy)-biphenyl (S form,m.p. 44.4 ° C)
4′-octyl-4-(2′-butanoyloxypropoxy)-biphenyl (S form, m.p. 50.7 - 50.8 ° C)
4′-octyl-4-(2′-pentanoyloxypropoxy)-biphenyl
4′-octyl-4-(2′-hexanoyloxypropoxy)-biphenyl (S form, m.p. 59.5 - 60.1 ° C)
4′-octyl-4-(2′-heptanoyloxypropoxy)-biphenyl (S form, m.p. 50.7 ° C)
4′-octyl-4-(2′-octanoyloxypropoxy)-biphenyl
4′-hexyloxy-4-(2′-butanoyloxypropoxy)-biphenyl (S form, m.p. 74.4 - 75.4 ° C)
4′-hexyloxy-4-(2′-hexanoyloxypropoxy)-biphenyl (S form, m.p. 75.1 - 76.0 ° C)
4′-hexyloxy-4-(2′-heptanoyloxypropoxy)-biphenyl (S form, m.p. 75.5 - 76.8 ° C)

4′-hexyloxy-4-(2′-nonanoyloxypropoxy)-biphenyl (S form, 79.2 - 79.5 ° C)
4′-hexyloxy-4-(2′-(4″-methylpentanoyloxy)-propoxy)-biphenyl
4′-hexyloxy-4-(2′-(4″-methylhexanoyloxy)-propoxy)-biphenyl (2′S, 4″S, m.p. 59.8 - 60.9 ° C)
4′-octyloxy-4-(2′-acetoxypropoxy)-biphenyl (S form, m.p. 81 ° C)
4′-octyloxy-4-(2′-propanoyloxypropoxy)-biphenyl (S form, m.p. 77.1 ° C)
4′-octyloxy-4-(2′-butanoyloxypropoxy)-biphenyl (S form, m.p. 70.9 ° C)
4-octyloxy-4-(2′-pentanoyloxypropoxy)-biphenyl (S form, m.p. 76.3 - 76.6 ° C)
4′-octyloxy-4-(2′-hexanoyloxypropoxy)-biphenyl (S form, m.p. 74.5 ° C)
4′-octyloxy-4-(2′-heptanoyloxypropoxy)-biphenyl (S form, m.p. 78.0 - 78.4 ° C)
4′-octyloxy-4-(2′-octanoyloxypropoxy)-biphenyl (S form, m.p. 78.8 - 79.3 ° C)
4′-octyloxy-4-(2′-nonanoyloxypropoxy)-biphenyl
4′-octyloxy-4-(2′-(2″-methylbutanoyloxy)-propoxy)-biphenyl (2′S, 2″S, m.p. 49 ° C)
4′-octyloxy-4-(2′-(4″-methylhexanoyloxy)-propoxy)-biphenyl (2′S, 4″S, m.p. 56 ° C)
4′-octyloxy-4-(2′-(3″-methylbutanoyloxy)-propoxy)-biphenyl (S form, m.p. 75 ° C)
4′-octyloxy-4-(2′-(4″-methylpentanoyloxy)-propoxy)-biphenyl (S form, m.p. 77 ° C)
4′-undecyloxy-4-(2′-propanoyloxypropoxy)-biphenyl (S form, m.p. 85.8 - 86.3 ° C)
4'-undecyloxy-4-(2′-pentanoyloxypropoxy)-biphenyl (S form, m.p. 82.7 - 83.9 ° C)
4′-undecyloxy-4-(2′-hexanoyloxypropoxy)-biphenyl
4′-undecyloxy-4-(2′-heptanoyloxypropoxy)-biphenyl (S form, m.p. 82.6 - 83.7 ° C)
4′-undecyloxy-4-(2′-octanoyloxypropoxy)-biphenyl (S form, m.p. 82.7 - 83.9 ° C)
4′-undecyloxy-4-(2′-(3″,-methylbutanoyloxy)-propoxy)-biphenyl (S form, m.p. 80.9 - 81.4 ° C)
4′-undecyloxy-4-(2′-(5″-methylheptanoyloxy)-propoxy)-biphenyl (2′S, 5″S, m.p. 65.4 - 66.5 ° C)
4′-pentyl-3-fluoro-4-(2′-propanoyloxypropoxy)-biphenyl
4′-pentyl-3-fluoro-4-(2′-pentanoyloxypropoxy)-biphenyl
4′-pentyl-3-fluoro-4-(2′-hexanoyloxypropoxy)-biphenyl
4′-pentyl-3-fluoro-4-(2′-heptanoyloxypropoxy)-biphenyl
3′-fluoro-4′-octyloxy-4-(2′-butanoyloxypropoxy)-biphenyl
3′-fluoro-4′-octyloxy-4-(2′-pentanoyloxypropoxy)-biphenyl
3′-fluoro-4′-octyloxy-4-(2′-hexanoyloxypropoxy)-biphenyl
3′-fluoro-4′-octyloxy-4-(2′-(5″-methylheptanoyloxy)propoxy)-biphenyl
4′-nonanoyl-3-fluoro-4-(2′-butanoyloxypropoxy)-biphenyl
4′-nonanoyl-3-fluoro-4-(2′-pentanoyloxypropoxy)-biphenyl
4′-nonanoyl-3-fluoro-4-(2′-hexanoyloxypropoxy)-biphenyl (S form, m.p. 79.7 - 80.2 ° C)
4′-nonanoyl-3-fluoro-4-(2′-(4″-methylhexanoyloxy)-propoxy)-biphenyl
4′-dodecanoyl-3-chloro-4-(2′-butanoyloxypropoxy)-biphenyl
4′-dodecanoyl-3-chloro-4-(2′-pentanoyloxypropoxy)-biphenyl
4′-dodecanoyl-3-chloro-4-(2′-heptanoyloxypropoxy)-biphenyl (S form, 43.7 - 43.9 ° C)
4′-dodecanoyl-3-chloro-4-(2′-(4″-methylhexanoyloxy)-propoxy)-biphenyl (2′S, 4″S, m.p. 43.2 - 43.4 ° C)
5-octyl-2-(4′-(2″-butanoyloxypropoxy)-phenyl)-pyrimidine (S form, m.p. 55 ° C)
5-octyl-2-(4′-(2″-pentanoyloxypropoxy)-phenyl)-pyrimidine (S form, m.p. 61 ° C)
5-octyl-2-(4′-(2″-heptanoyloxypropoxy)-phenyl)-pyrimidine (S form, m.p. 59 ° C)
2-(4′-octylphenyl)-5-(2′-butanoyloxypropoxy)-pyrimidine (S form, m.p. 34.5 - 35.9 ° C)
2-(4′-octylphenyl)-5-(2′-pentanoyloxypropoxy)-pyrimidine (S form, m.p. 32.8 - 33.3 ° C)
2-(4′-octylphenyl)-5-(2′-hexanoyloxypropoxy)-pyrimidine (S form, m.p. 36.7 - 38.4 ° C)
2-(4′-octylphenyl)-5-(2′-heptanoyloxypropoxy)-pyrimidine (S form, m.p. 38.1 - 39.0 ° C)
2-(4′-octylphenyl)-5-(2′-(4″-methylhexanoyloxy)-propoxy)-pyrimidine (2′S, 4″S, m.p. 33.0 - 34.3 ° C)
2-(4′-octylphenyl)-5-(2′-(3″-methylbutanoyloxy)-propoxy)-pyrimidine (S form, m.p. 42.5 - 42.7 ° C)
5-octyl-2-(3′-fluoro-4′-(2″-pentanoyloxypropoxy)-phenyl)-pyrimidine (S form, m.p. 34.1 ° C)
5-octyloxy-2-(4′-(2″-butanoyloxypropoxy)-phenyl)-pyridine
5-octyloxy-2-(4′-(2″-pentanoyloxypropoxy)-phenyl)-pyrimidine
5-octyloxy-2-(4′-(2″-octanoyloxypropoxy)-phenyl)-pyrimidine
5-nonyl-2-(4′-(2″-butanoyloxypropoxy)-phenyl)-pyridine (S form, m.p. 46.5 ° C)
5-nonyl-2-(4′-(2″-pentanoyloxypropoxy)-phenyl)-pyridine (S form, m.p. 45.2 ° C)
5-nonyl-2-(4′-(2″-heptanoyloxypropoxy)-phenyl)-pyridine (S form, m.p. 48.7 ° C)
5-octyl-2-(3′-fluoro-4′-(2″-pentanoyloxypropoxy)-phenyl)-pyridine
3-(4′-octyloxyphenyl)-6-(2′-butanoyloxypropoxy)-pyridazine (S form, m.p. 72.7 - 73.1 ° C)
3-(4′-octyloxyphenyl)-6-(2′-pentanoyloxypropoxy)-pyridazine (S form, m.p. 67.2 - 68.1 ° C)
3-(4′-octyloxyphenyl)-6-(2′-heptanoyloxypropoxy)-pyridazine (S form, m.p. 56.5 - 56.7 ° C)

3-(4′-nonylphenyl)-6-(2′-propanoyloxypropoxy)-pyridazine (S form, m.p. 81.5 - 82.6 ° C)
3-(4′-nonylphenyl)-6-(2′-pentanoyloxypropoxy)-pyridazine (S form, m.p. 87.0 ° C)
3-(4′-nonylphenyl)-6-(2′-hexanoyloxypropoxy)-pyridazine (S form, m.p. 90.2 - 92.0 ° C)
2-(4′-octyloxyphenyl)-5-(2′-butanoyloxypropoxy)-pyrazine
2-(4′-octyloxyphenyl)-5-(2′-pentanoyloxypropoxy)-pyrazine
2-(4′-octyloxyphenyl)-5-(2′-heptanoyloxypropoxy)-pyrazine
1-(4′-pentyl-trans-cyclohexyl)-4-(2′-butanoyloxypropoxy)-benzene (S form, m.p. 28.4 ° C)
1-(4′-pentyl-trans-cyclohexyl)-4-(2′-pentanoyloxypropoxy)-benzene
1-(4′-pentyl-trans-cyclohexyl)-4-(2′-hexanoyloxypropoxy)-benzene (S form, m.p. 21.4 ° C)
4′-heptyl-4-(2′-(2″-ethoxyacetoxy)-propoxy)-biphenyl (S form, m.p. 59.9 - 60.3 ° C)
4′-octyl-4-(2′-(2″-methoxyacetoxy)-propoxy)-biphenyl
4′-octyloxy-4-(2′-(2″-ethoxyacetoxy)-propoxy)-biphenyl (S form, m.p. 84.6 - 85.0 ° C)
4′-octyloxy-4-(2′-(3″-ethoxypropanoyloxy)-propoxy)-biphenyl (S form, m.p. 57.5 - 57.7 ° C)
5-nonyl-2-(4′-(2″-(3″-ethoxypropanoyloxy)-propoxy)-phenyl)-pyridine (S form, m.p. 40.0 - 40.4 ° C)
5-nonyl-2-(4′-(2″-(2″-ethoxyacetoxy)-propoxy)-pehnyl)-pyridine (S form, m.p. 47.7 - 48.6 ° C)
5-octyl-2-(4′-(2″-(2″-methoxyacetoxy)-propoxy)-phenyl)-pyrimidine (S form, m.p. 45.6 - 47.0 ° C)
5-octyloxy-2-(4′-(2″-(3″-methoxypropanoyloxy)-propoxy)-phenyl)-pyrimidine
2-(4′-octylphenyl)-5-(2′-(2″-ethoxyacetoxy)-propoxy)-pyrimidine (S form, m.p. 47.7 - 48.6 ° C)
2-(4′-heptyloxyphenyl)-5-(2′-(3″-methoxypropanoyloxy)-propoxy)-pyrimidine
1-(4′-pentyl-trans-cyclohexyl)-4-(2′-(2″-ethoxyacetoxy)-propoxy)-benzene
3-(4′-octyloxyphenyl)-6-(2′-(3″-ethoxypropanoyloxy)-pyridazine (S form, m.p. 58.5 - 59.0 ° C)
2-(4′-octyloxyphenyl)-5-(2′-(2″-methoxyacetoxy)-propoxy)-pyradine
4-butyl-4-(2′-(4″-keto-pentanoyloxy)-propoxy)-biphenyl
4′-hexyl-4-(2′-(5″-keto-hexanoyloxy)-propoxy)-biphenyl
4′-nonyl-4-(2′-(5″-keto-heptanoyloxy)-propoxy)-biphenyl
4′-octyloxy-4-(2′-(4″-keto-pentanoyloxy)-propoxy)biphenyl (S form, m.p. 80.5 ° C)
4′-octyloxy-4-(2′-(5″-keto-hexanoyloxy)-propoxy)-biphenyl (S form, m.p. 89.1 ° C)
5-octyl-2-(4′-(2″-(4‴-keto-pentanoyloxy)-propoxy)-phenyl)-pyridine
5-nonyl-2-(4′-(2″-(4‴ -keto-pentanoyloxy)-propoxy)-phenyl)-pyridine (S form, m.p. 49.3 ° C)
5-nonyl-2-(4′-(2″-(5‴ -keto-hexanoyloxy)-propoxy)-phenyl)-pyridine
5-octyl-2-(4′-(2″-(4‴ -keto-pentanoyloxy)-propoxy)-phenyl)-pyrimidine
5-octyloxy-2-(4′-(2″-(5‴ -keto-hexanoyloxy)-propoxy)-phenyl)-pyrimidine
2-(4′-octylphenyl)-5-(2′-(4″-keto-pentanoyloxy)-propoxy)-pyrimidine
2-(4′-heptyloxyphenyl)-5-(2′-(5″-keto-hexanoyloxy)-propoxy)-pyrimidine
1-(4′-pentyl-trans-cyclohexyl)-4-(2′-(4″-keto-pentanoyloxy)-propoxy)-benzene
3-(4′-octyloxyphenyl)-6-(2′-(5″-keto-hexanoyloxy)-propoxy)-pyridazine
2-(4′-octyloxyphenyl)-5-(2′-(4″-k-to-pentanoyloxy)-propoxy)-pyridine
4′-octyloxy-4-(3′-butanoyloxybutoxy)-biphenyl (S form, m.p. 57.4 - 57.7 ° C)
4′-octyloxy-4-(3′-pentanoyloxybutoxy)-biphenyl (R form, m.p. 50.0 ° C)
4′-octyloxy-4-(3′-heptanoyloxybutoxy)-biphenyl (R form, m.p. 56.4 - 56.8 ° C)
2-(4′-octylphenyl)-5-(3′-pentanoyloxybutoxy)-pyrimidine (R form, oily at room temperature)
2-(4′-octylphenyl)-5-(3′-hexanoyloxybutoxy)-pyrimidine (R form, oily at room temperature)
5-octyl-2-(4′-(3″-propanoyloxybutoxy)-phenyl)-pyrimidine
5-octyl-2-(4′-(3″-heptanoyloxybutoxy)-phenyl)-pyrimidine
5-octyloxy-2-(4′-(3″-butanoyloxybutoxy)-phenyl)-pyrimidine
5-octyloxy-2-(4′-(3″-hexanoyloxybutoxy)-phenyl)-pyrimidine
5-octyl-2-(4′-(3″-butanoyloxybutoxy)-phenyl)-pyridine (R form, m.p. 44.7 - 45.6 ° C)
5-octyl-2-(4′-(3″-pentanoyloxybutoxy)-phenyl)-pyridine (R form, m.p. 46.5 - 47.5 ° C)
5-octyl-2-(4′-(3″-hexanoyloxybytoxy)-phenyl)-pyridine (R form, m.p. 49.9 -50.4 ° C)
1-(4′-pentyl-trans-cyclohexyl)-4-(3″-pentanoyloxybutoxy)-benzene
3-(4′-octylphenyl)-6-(3′-hexanoylbutoxy)-pyridazine
2-(4′-octyloxyphenyl)-5-(3′-butanoyloxybutoxy)-pyrazine
4′-octyloxy-4-(4′-butanoyloxypentoxy)-biphenyl
2-(4′-octylphenyl)-5-(4′-hexanoylpentoxy)-pyrimidine
5-octyl-2-(4′-(4″-pentanoyloxypentoxy)-phenyl)-pyrimidine
5-octyloxy-2-(4′-(4″-heptanoyloxypentoxy)-phenyl)-pyrimidine
5-octyl-2-(4′-(4″-butanoyloxypentoxy)-phenyl)-pyrimidine
1-(4′-hexyl-trans-cyclohexyl)-4-(4′-heptanoyloxypentoxy)-benzene

3-(4′-octyloxyphenyl)-6-(3′-propanoyloxypentoxy)-pyridazine
2-(4′-octylphenyl)-5-3′-octanoyloxypentocy)-pyrazine
4′-heptyl-4-(5′propanoyloxyhexyloxy)-biphenyl
2-(4′-octyloxyphenyl)-5-(6′-butanoyloxyheptyloxy)-pyrimidine
5-octyl-2-(4′-(7″-hexanoyloxyoctyloxy)-phenyl)-pyridine
1-(4′-octyl-trans-cyclohexyl)-4-(8′-heptanoyloxynonyloxy)-benzene
3-(4′-octyloxyphenyl)-6-(9′-butanoyloxydecyloxy)-pyridazine
2-(4′-octyloxyphenyl)-5-(10′-propanoyloxyundecyloxy)-pyrazine

The compounds and liquid crystal compositions of the present invention will be described below in more detail by way of Examples.

Example 1

Preparation of S-4′-octyloxy-4-(2′-pentanoyloxypropoxy)-biphenyl

(a compound of the formula (I) wherein $R^1$ represents $C_8H_{17}O$; $R^2$ represents $C_4H_9$; 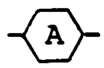 and 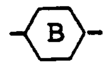 both represent 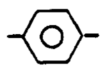; m represents 1; and n represents 1)

A mixture of (2S)-2-tetrahydropyranyloxy-1-hydroxypropane (137 g, 0.85 mol) prepared according to the method described in a literature (C. Malanga et al, Synthetic Communications, 12 (1), 67-70 (1982)), with anhydrous pyridine (600 g) was cooled with ice, followed by dropwise adding to the mixture a solution of p-toluenesulfonyl chloride (165 g, 0.87 mol) dissolved in pyridine (200 mℓ), agitating the mixture at 0 °C for 2 hours, successively agitating it at room temperature for 2 hours, allowing it to stand overnight, adding toluene (1 ℓ), further adding 2N-NaOH aqueous solution (500 mℓ), separating the resulting organic layer, several times washing it with water to make it neutral, drying with $MgSO_4$ and distilling off the solvent to obtain (2S)-2-(2′-tetrahydropyranyloxy)-1-(p-toluenesulfonyloxy)-propane (257 g, yield 95.9%). A solution of this product (20 g) dissolved in N,N-dimethylformamide (hereinafter abbreviated to DMF) (300 mℓ) was added to a mixture of sodium hydride (60%, 2 g), 4-hydroxy-4′-octyloxy-biphenyl (10 g) and tetrahydrofuran (hereinafter abbreviated to THF) (200 mℓ) followed by agitating the mixture at 60 °C for 4 hours, allowing it to cool down to room temperature, adding toluene (300 mℓ) and water (300 mℓ), separating the resulting organic layer, washing it with an alkali, washing with water, concentrating, assing ethanol (300 mℓ) and pyridium p-toluenesulfonate (hereinafter abbreviated to PPTS) (2 g), agitating the mixture at 560 °C for 3 hours, distilling off ethanol, adding toluene (300 mℓ), washing the resulting organic layer with water, concentrating it and recrystallizing to obtain S-1-(4′-octyloxy-4-biphenylyloxy)-propan-2-ol (8 g). A mixture of this product (1.5 g), pentanoylchloride (0.8 g) and pyridine (50 mℓ) was heated to 60 °C for 2 hours. After the reaction, the resulting material was purified to obtain the objective S-4′-octyloxy-4-(2′-pentanoyloxypropoxy)-biphenyl (0.8 g). m.p.: 76.3 -76.6 °C.

Example 2

Preparation of S-4′-octyloxy-4-(2′-butoxycarbonyloxypropoxy)-biphenyl

(a compound of the formula (I) wherein $R^1$ represents $C_8H_{17}O$, $R^2$ represents $C_4H_9O$, 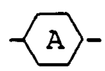 and 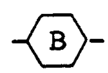 both represent 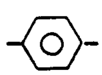, m represents 1 and n represents 1)

A mixture of S-1-(4′-octyloxy-4-biphenylyloxy)-propan-2-ol 91.5 g) obtained as an intermediate in the

steps of Example 1, butyl chloroformate (0.9 g) and pyridine (10 mℓ) was reacted in the same manner as in Example 1, followed by purification to obtain S-4′-octyloxy-4-(2′-butoxycarbonyloxypropoxy)-biphenyl (0.7 g). m.p.: 66.9 -67.8 ° C.

Example 3

Preparation of S-4′-octyloxy-4-(2′-heptanoyloxypropoxy)-biphenyl

(a compound of the formula (I) wherein $R^1$ represents $C_8H_{17}O$; $R^2$ represents $C_6H_{13}$; 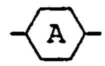 and 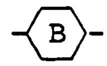 both represent 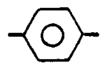; m represents 1; and n represents 1)

S-1-(4′-octyloxy-4-biphenylyloxy)-propan-2-ol (1.5 g) obtained as an intermediate in the preparation of Example 1, heptanoic acid (0.5 g), N,N-dicyclohexylcarbodiimide (hereinafter abbreviated to DCC) (1.3 g) and 4-N,N-dimethylaminopyridine (hereinafter abbreviated to DMAP) (0.1 g) were agitated in dichloromethane (50 mℓ) at room temperature for 4 hours, followed by filtering off the resulting solids and purifying the mother liquor to obtain the captioned compound (m.p. 78.0 - 78.4 ° C).

Example 4

Preparation of (2′S, 2″S)-4′-octyloxy-4-(2′-(2″-methylbutylyloxy)-propoxy)-biphenyl

(a compound of the formula (I) wherein $R^1$ represents $C_8H_{17}O$; $R^2$ represents $-\overset{CH_3}{\underset{*}{C}}HCH_2CH_3$; 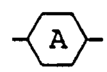 and 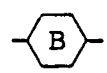 both represent 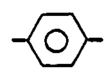; m represents 1; and n represents 1)

Example 3 was repeated except that heptanoic acid in Example 3 was replaced by S-2-methylbutanoic acid, to obtain the captioned compound (m.p. 49 ° C).

Example 5 (Composition 1)

A liquid crystal composition A consisting of

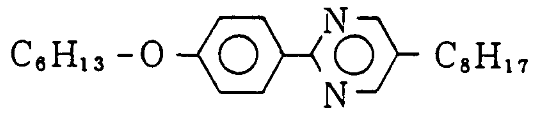 30 wt.%

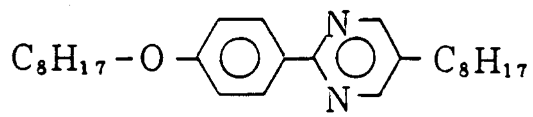 20 wt.%

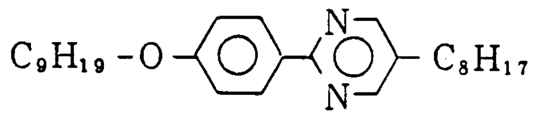 10 wt.%

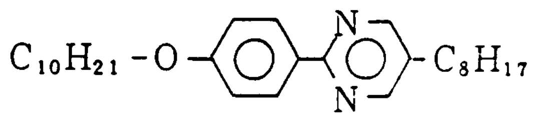 10 wt.%

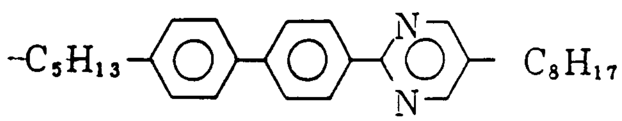 20 wt.% and

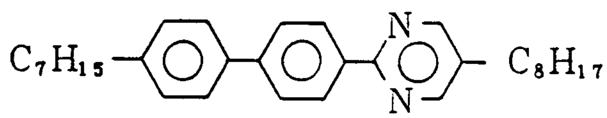 10 wt.%

exhibits the following phase transition points:
C → SC: 4 ° C, SC → SA: 65 ° C, SA → N: 79 ° C and N → I: 90 ° C wherein C, SA, N and I are abbreviations of crystalline phase, smectic A phase, nematic phase and isotropic liquid, respectively. Further, since this composition consists only of non-optically active compounds, it is not a chiral liquid crystal and hence exhibits no spontaneous polarization.

A composition B consisting of 80% by weight of the above composition A and 20% by weight of the compound of Example 1 of the present invention exhibits the following phase transition points:
SC* → SA: 46 ° C, SA → Ch: 73.8 ° C and Ch → I: 80.2 ° C, although the phase transition point of C → SC* is unclear.

This composition B was sealed in a cell of 2 µm thick provided with transparent electrodes obtained by applying PVA (polyvinyl alcohol) as an aligning agent onto the surface and rubbing the resulting surface to subject it to parallel aligning treatment, followed by providing the resulting element between two crossed polarizers and impressing an electric field thereto. As a result, change in the intensity of transmitted light was observed by impressing ±10V. Response time was sought from the change in the intensity of transmitted light at that time and the spontaneous polarization value Ps was sought according to Sowyer-Tower method. The results were as follows:

| Temperature (° C) | Response time (µsec) | Ps (nC/cm$^2$) |
|---|---|---|
| 40 | 18 | 5.7 |
| 35 | 28 | 8.5 |
| 30 | 36 | 10.1 |
| 25 | 48 | 11.3 |

As shown above, when the compound of the formula (I) of the present invention was used, it was possible to impart Ps to an achiral smectic composition and a ferroelectric liquid crystal composition exhibiting a response time of about 50 µsec at room temperature was obtained.

Example 6 (Composition 2)

A liquid crystal composition C consisting of

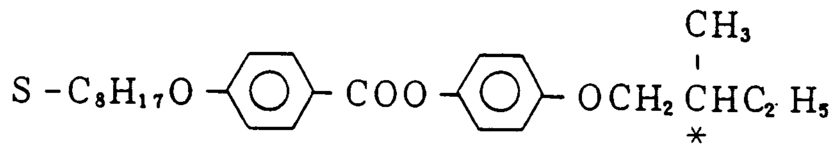

40 wt. %

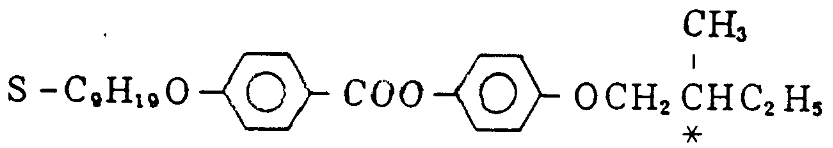

30 wt. % and

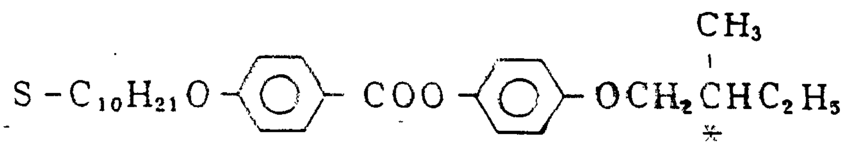

30 wt. %

exhibits the following phase transition points:
C → SB: 17 °C, SB → SC*: 22 °C, SC* → SA: 45 °C and SA → I: 59 °C.

A liquid crystal composition D obtained by adding 20% by weight of the compound of Example 1 of the present invention to 80% by weight of the composition C exhibited the following phase transition points:
SC* → SA: 36 °C and SA → I: 53 °C
although the phase transition point of C → SC* was unclear.

The response times and Pss of the compositions C and D were measured under the same conditions as in Example 5 (Composition 1). The results were as follows:

| Composition | Temperature (°C) | Response time (μsec) | Ps (nC/cm²) |
|---|---|---|---|
| C | 25 | 2300 | 4.0 |
| D | 30 | 81 | 26.4 |
| D | 25 | 125 | 35.9 |
| D | 20 | 196 | 42.5 |

As shown above, when the compound of the formula (I) of the present invention is added to a composition which is a chiral smectic liquid crystal composition, but has a small Ps value and a low response rate, it is possible to increase the Ps value of the composition and reduce the response time down to a value as small as about 1/20.

Example 7 (Composition 3)

A mixture (liquid crystal composition E) consisting of 90% by weight of the liquid crystal composition A used in Example 5 (Composition 1) and 10% by weight of S-5-nonyl-2-(4'-(2"-butanoyloxypropoxy)-phenyl)-pyridine (m.p. 46.5 °C) (a compound of the present invention) exhibits the following phase transition points:
SC* → SA, 46.5 °C; SA → Ch, 73.8 °C; and Ch → I, 82.5 °C.

The response time and Ps of the liquid crystal composition E were measured under the same

conditions as in Example 5 (Composition 1). The results are as follows:

| Temperature (° C) | Response time (μsec) | Ps (nC/cm²) |
|---|---|---|
| 40 | 50 | 3.3 |
| 30 | 85 | 4.3 |
| 20 | 110 | 4.7 |

As shown above, when 10% by weight of the compound of the present invention is used, the resulting composition has a sufficiently short response time.

<u>Example 8 (Composition 4)</u>

A nematic liquid crystal composition consisting of

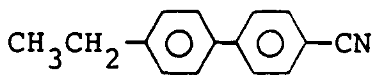 20 wt.%

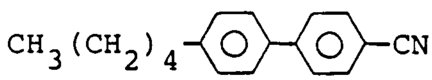 35 wt.%

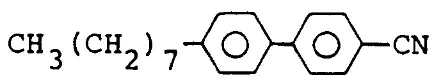 30 wt.% and

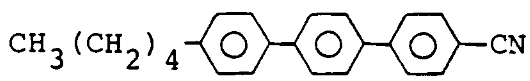 15 wt.%

was filled in a cell provided with transparent electrodes obtained by applying polyvinyl alcohol (PVA) as an aligning agent onto the surfaces thereof and rubbing the resulting surfaces to subject them to parallel aligning treatment and having a distance between the electrodes of 10 μm to prepare a TN display cell, which was observed under a polarizing microscope . As a result, formation of a reverse twist domain was observed.

To this nematic liquid crystal composition was added (S)-5-octyl-2-(4′-(2″-butanoyloxypropoxy)-phenyl)-pyrimidine in 0.5% by weight, and the resulting TN type cell was observed in the same manner as above. As a result no reverse twist domain formed, but a uniform nematic phase was observed.

<u>Example 9 (Composition 5)</u>

(S)-3-(4′-nonylphenyl)-6-(2′-pentanoyloxypropoxy)-pyridazine as a compound of the present invention in 1% by weight was added to a commercially available nematic liquid crystal composition (ZLI-1132, tradename of a product of Merck Co., Ltd.) to obtain a chiral nematic liquid crystal composition the chiral pitch of which was then measured according to Cano wedge method (Applied Physics <u>43</u> (2), 126-131 (1974)). The composition exhibited the following negative temperature characteristics:

| Temperature | Pitch (μm) |
|---|---|
| 20 | 15.2 |
| 30 | 14.7 |
| 40 | 14.3 |
| 50 | 14.1 |
| 60 | 14.1 |

**Claims**

**1.** Compounds of the formula:

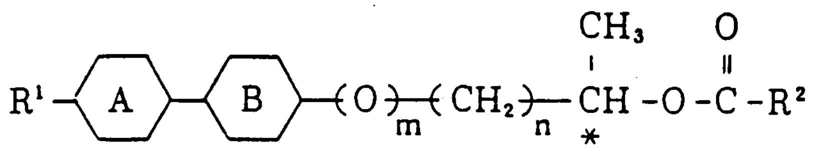 (I)

in which $R^1$ is a straight or branched alkyl, alkoxy, alkanoyl, alkanoyloxy, alkoxycarbonyl, alkoxycarbonyloxy or alkoxyalkyl group, each containing from 1 to 20 carbon atoms; $R^2$ is a hydrogen atom or a straight or branched alkyl, alkoxy ω-halo-substituted alkyl, cyano thioalkyl, alkoxy, alkanoyl, alkanoyloxy, alkoxycarbonyl or alkoxycarbonyloxy group, each containing from 1 to 15 carbon atoms;

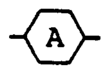 and 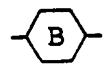

are the same or are different and each is a group:

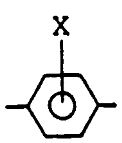, 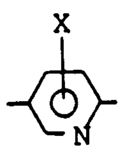, 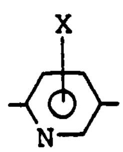, 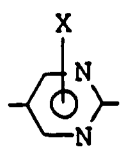, 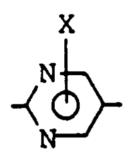, 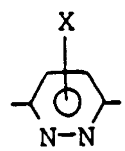,

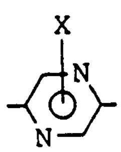 or 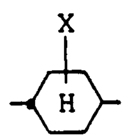

(in which X is a hydrogen, fluorine chlorine atom or a cyano, methyl or trifluoromethyl group); m is 0 or 1; n is a natural number of 1 to 10; and the symbol * indicates that the carbon atom to which the symbol is attached is an asymmetric carbon atom.

**2.** Compounds as claimed in claim 1 wherein

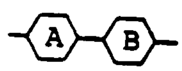

is a grouping

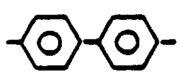, 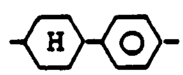, 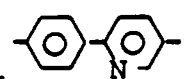,

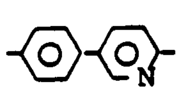, 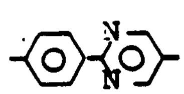, 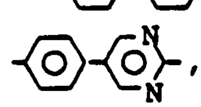, 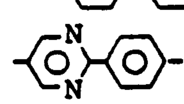, 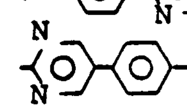,

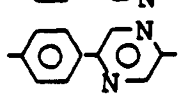 or 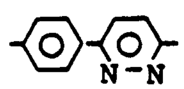.

3. Compounds as claimed in claim 2 wherein either one of the groups

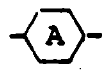 or 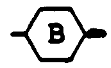

is substituted with a fluorine atom or a cyano or methyl group.

4. Compound as claimed in any one of the preceding claims in which $R^1$ contains from 4 to 15 carbon atoms and $R^2$ contains from 1 to 8 carbon atoms.

5. Compound as claimed in claim 4 in which $R^1$ contains from 6 to 12 carbon atoms and $R^2$ contains from 1 to 6 carbon atoms.

6. Compounds as claimed in any one of the preceding claims in which $\underline{m}$ is 1 $\underline{n}$ is a natural number of from 1 to 6.

7. A liquid crystal composition comprising at least two components at least one of which is a compound as claimed in any one of the preceding claims.

8. A liquid crystal composition as claimed in claim 7 which exhibits a chiral smectic phase.

9. A liquid crystal composition as claimed in claim 7 which exhibits a chiral nematic phase.

10. A light-switching element comprising a liquid crystal composition as claimed in claim 7.

**Revendications**

1. Composés de la formule:

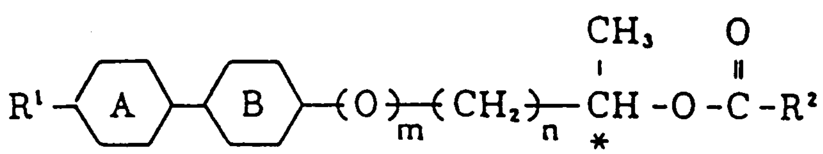 (I)

dans laquelle $R^1$ est un groupement alkyle, alcoxy, alcanoyle, alcanoyloxy, alcoxycarbonyle, alcoxycarbonyloxy ou alcoxyalkyle à chaîne droite ou ramifiée, contenant chacun de 1 à 20 atomes de carbone; $R^2$ est un atome d'hydrogène ou un groupement à chaîne droite ou ramifiée alkyle, alcoxy, alkyle substitué en position $\omega$ par un halogène, un groupement cyano, thioalkyle, alcoxy, alcanoyle, alcanoyloxy, alcoxycarbonyle ou alcoxycarbonyloxy, contenant chacun de 1 à 15 atomes de carbone;

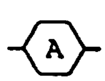 et 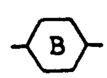

sont identiques ou différents et chacun d'eux est un groupement:

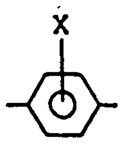, 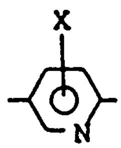, 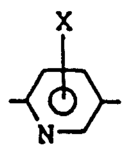, 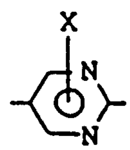, 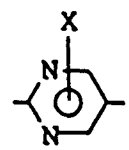, 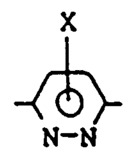,

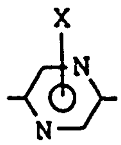 ou 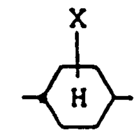

(dans lesquelles X est un atome d'hydrogène, de fluor ou de chlore ou un groupement cyano, méthyle ou trifluorométhyle); m est 0 ou 1; n est un nombre entier de 1 à 10; et le symbole * indique que l'atome de carbone auquel le symbole est attaché est un atome de carbone asymétrique.

2. Composés tels que revendiqués dans la revendication 1, dans lesquels

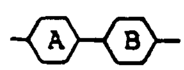

est un groupement

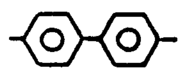, 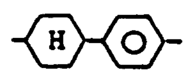, 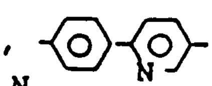,
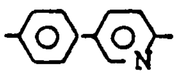, 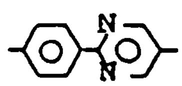, 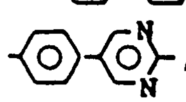, 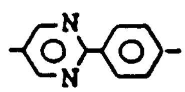, 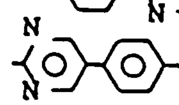,
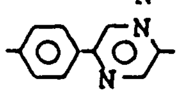 ou 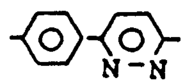.

3. Composés tels que revendiqués dans la revendication 2, dans lesquels l'un ou l'autre des groupements

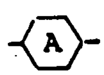 ou 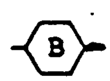

est substitué par un atome de fluor ou un groupement cyano ou méthyle.

4. Composés tels que revendiqués dans l'une quelconque des revendications précédentes, dans lesquels $R^1$ contient de 4 à 15 atomes de carbone et $R^2$ contient de 1 à 8 atomes de carbone.

5. Composés tels que revendiqués dans la revendication 4, dans lesquels $R^1$ contient de 6 à 12 atomes de carbone et $R^2$ contient de 1 à 6 atomes de carbone.

6. Composés tels que revendiqués dans l'une quelconque des revendications précédentes, dans lesquels m est 1 et n est un nombre entier de 1 à 6.

7. Une composition de cristaux liquides comprenant au moins deux composants dont au moins un est un composé tel que revendiqué dans l'une quelconque des revendications précédentes.

8. Une composition de cristaux liquides telle que revendiquée dans la revendication 7 qui présente une phase smectique chirale.

9. Une composition de cristaux liquides telle que revendiquée dans la revendication 7, qui présente une phase nématique chirale.

10. Un élément de commutation de lumière comprenant une composition de cristaux liquides telle que revendiquée dans la revendication 7.

**Patentansprüche**

1. Verbindungen der Formel:

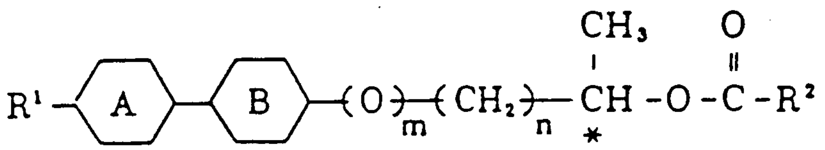

(I)

worin bedeuten:

$R^1$ eine gerade (unverzweigte) oder verzweigte Alkyl-, Alkoxy-, Alkanoyl-, Alkanoyloxy-, Alkoxycarbonyl-, Alkoxycarbonyloxy- oder Alkoxyalkyl-Gruppe, die jeweils 1 bis 20 Kohlenstoffatome enthält;

$R^2$ ein Wasserstoffatom oder eine gerade (unverzweigte) oder verzweigte Alkyl-, Alkoxy-, ω-Halogen-substituierte Alkyl-, Cyano-, Thioalkyl-, Alkoxy-, Alkanoyl-, Alkanoyloxy-, Alkoxycarbonyl- oder Alkoxycarbonyloxy-Gruppe, die jeweils 1 bis 15 Kohlenstoffatome enthält,

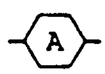 und 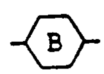 ,

die gleich oder verschieden sind, jeweils stehen für eine Gruppe

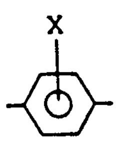 , 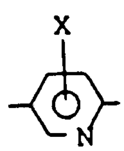 , 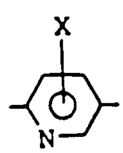 , 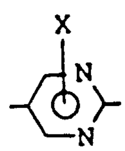 , 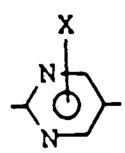 , 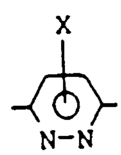 ,

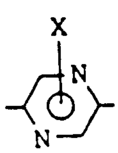 oder 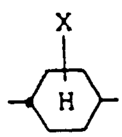

(worin X für ein Wasserstoff-, Fluor- oder Chloratom oder für eine Cyano-, Methyl- oder Trifluoromethyl-Gruppe steht);

m die Zahl 0 oder 1;

n eine natürliche Zahl von 1 bis 10; und

das Symbol * anzeigt, daß das Kohlenstoffatom, an dem das Symbol angebracht ist, ein asymmetrisches Kohlenstoffatom ist.

2. Verbindungen nach Anspruch 1, worin

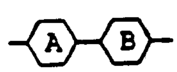

steht für eine Gruppierung:

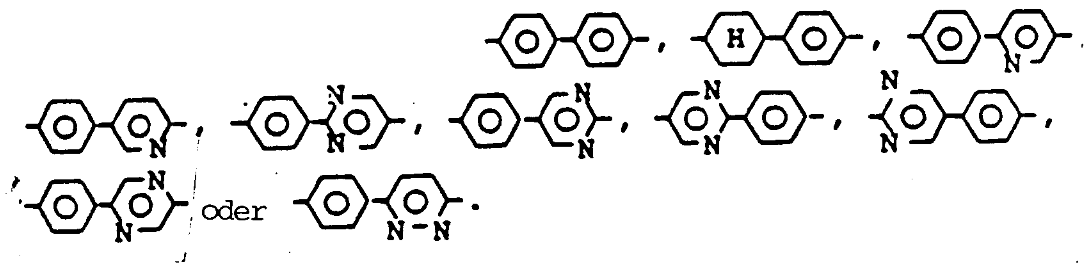

oder .

3. Verbindungen nach Anspruch 2, worin jeweils eine der Gruppen

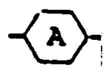 oder 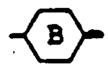

durch ein Fluoratom oder eine Cyano- oder Methylgruppe substituiert ist.

4. Verbindung nach einem der vorhergehenden Ansprüche, worin $R^1$ 4 bis 15 Kohlenstoffatome und $R^2$ 1 bis 8 Kohlenstoffatome enthalten.

5. Verbindung nach Anspruch 4, worin $R^1$ 6 bis 12 Kohlenstoffatome und $R^2$ 1 bis 6 Kohlenstoffatome enthalten.

6. Verbindungen nach einem der vorhergehenden Ansprüche, worin m für die Zahl 1 und n für eine natürliche Zahl von 1 bis 6 stehen.

7. Flüssigkristall-Zusammensetzung, die mindestens zwei Komponenten umfaßt, von denen mindestens eine eine Verbindung nach einem der vorhergehenden Ansprüche ist.

8. Flüssigkristall-Zusammensetzung nach Anspruch 7, die eine chirale smektische Phase aufweist.

9. Flüssigkristall-Zusammensetzung nach Anspruch 7, die eine chirale nematische Phase aufweist.

10. Lichtschalter-Element, das eine Flüssigkristall-Zusammensetzung nach Anspruch 7 umfaßt.